# EUROPEAN PATENT APPLICATION

(11) **EP 0 672 416 A1**
(43) Date of publication of application: **20.09.1995**
(21) Application number: 95301337.2
(22) Date of filing: 02.03.1995
(51) Int. Cl.: A61K 31/485, A61K 9/16

(54) **Pharmaceutical composition comprising diamorphine**

(30) Priority: 14.03.1994 GB 9404928; 14.06.1994 GB 9411842; 17.11.1994 EP 94308493; 21.11.1994 GB 9423498
(71) Applicant: Euro-Celtique S.A., Luxemburg (LU)
(72) Inventor: Miller, Ronald Brown, CH-4051 Basle (CH); Leslie, Stewart Thomas, Cambridge CB2 2RA (GB); Prater, Derek Allan, Milton, Cambridge CB4 4YS (GB); Knott, Trevor John, Wickford, Essex SS11 8YA (GB); Mohammad, Hassan, Haslingfield, Cambridge CB3 7LG (GB)
(74) Representative: Lamb, John Baxter

(57) **Abstract**

A controlled release pharmaceutical preparation, particularly for oral administration, comprises diamorphine, or a pharmaceutically acceptable salt thereof, as active ingredients, preferably in admixture with a hydrophobic fusible carrier or diluent.

## Description

This invention relates to controlled release preparations containing pharmaceutically active ingredients and in particular a controlled release preparation for oral use containing diamorphine or a pharmaceutically acceptable salt thereof.

Diamorphine is an opiate prescribed for severe pain. The currently used pharmaceutical formulations in which it is an active ingredient include injections, elixers, linctus, and powders (see Martindale, The Extra Pharmacopoeia 30th Ed.). Because of its instability in the presence of water the injection, elixers and linctus must each be freshly prepared before use, and the powders are to be added to water immediately before use. Conventional granulating techniques for the preparation of controlled release preparations are not possible because of the rapid degradation of diamorphine in the presence of water and consequently formulating with release control components to provide dosage forms such as capsules containing granules, or tablets formed from compressed granules by such techniques has not, hitherto, been possible. All known pharmaceutical preparations of this substance provide only for instant release.

It is desirable that there should be available to patients suffering from severe pain a diamorphine preparation which has reasonable storage stability, does not have to be freshly prepared before use, is easily self administered and which has a duration of activity of 12 hours or more, preferably 24 hours, and accordingly needs to be administered only twice or one-a-day.

An additional need for such a formulation has existed for some years in the field of the treatment of drug addiction. It is the policy in certain countries to provide registered addicts with free access under controlled conditions, to diamorphine (heroin); the aim of such a policy being to remove such addicts as customers from the black market; but also to reduce the otherwise widespread practice of needle sharing which has resulted in a rapid spread of HIV and hepatitis infection among addicts. Nonetheless, such a policy presently requires the provision of diamorphine for self injection by addicts and requires considerable control and supervision. There is, therefore, a need for a controlled release diamorphine preparation which would facilitate treatment according to such a policy.

In its broadest aspect the present invention provides a controlled release, pharmaceutical preparation which comprises diamorphine or a pharmaceutically acceptable salt thereof as an active ingredient.

The preparation of the invention is preferably in the form of a tablet or multiparticulates comprising the diamorphine or pharmaceutically acceptable salt thereof incorporated in a controlled release matrix, which matrix preferably comprises a hydrophobic, fusible carrier or diluent in which the diamorphine or salt thereof is incorporated.

A pharmaceutical preparation in accordance with the invention containing diamorphine or a salt thereof as an active ingredient suitable for once or twice-a-day dosing which is preferably obtained by incorporation of the diamorphine or salt in a matrix system comprising a hydrophobic, fusible binder and optionally a release modifying component may preferably have an in vitro dissolution rate as measured by modified Ph. Eur. basket or Ph. Eur. Paddle method at 100rpm in 900ml aqueous buffer at pH 4.5 at 37°C (phosphate or acetate buffer) as follows:

| **TIME (HR)** | **% DIAMORPHINE RELEASED** |
|---|---|
| 1 | 0-70 |
| 2 | <80 |
| 4 | 10-100 |
| 12 | 40-100 |
| 16 | 50-100 |

One preferred formulation in accordance with the invention has a dissolution rate as follows:-

| **TIME (HR)** | **% DIAMORPHINE RELEASED** |
|---|---|
| 1 | 10-70 |
| 2 | 15-80 |
| 4 | 30-100 |
| 6 | 40-100 |
| 12 | 60-100 |
| 16 | 70-100 |

Yet another preferred formulation in accordance with the invention has a dissolution rate as follows:

| **TIME (HR)** | **% DIAMORPHINE RELEASED** |
|---|---|
| 1 | 0-50 |
| 4 | 10-80 |
| 8 | 30-100 |
| 12 | 40-100 |
| 16 | 50-100 |

The controlled release preparation in accordance with the invention can, surprisingly, be produced without the use of water in a granulation step, or other process features leading to degradation of the diamorphine. As a result the preparations in accordance with the invention are not only pharmaceutically acceptable but the products possess two important advantages in that they show long term stability on storage, and also the dissolution rates are stable over a long period of time.

A preferred preparation in accordance with the invention shows, subject to experimental error, no change in diamorphine salt content, nor in 6-0-acetylmorphine (or salt) or morphine (or salt) content nor any change in in vitro dissolution rate (as defined herein) after storage for three months and more preferably six months at 30°C.

The pharmaceutical preparation in accordance with the invention in one preferred form comprises multiparticulates which generally are spherical or spheroidal particles of a size capable of passing through a mesh of size 0.1 - 3.0mm preferably 0.1 - 2.0mm, the multiparticulates preferably comprising a matrix of a hydrophobic, fusible release control material within which the diamorphine or a pharmaceutically acceptable salt thereof is dispersed.

The preparation may comprise a capsule e.g. a hard capsule, containing multiparticulates as described above; another, preferred preparation may comprise a tablet comprising compressed multiparticulates, granulates or controlled release seeds or particles.

We have found that the total amount of active ingredient in the composition may vary within wide limits, for example from 10 to 75% by weight thereof.

The hydrophobic, fusible component should be a hydrophobic material such as a natural or synthetic wax or oil, for example hydrogenated vegetable oil or hydrogenated castor oil, and suitably has a melting point of from 35 to 150°C.

A release modifying component, when a water soluble fusible material, is conveniently a polyethylene glycol and, when a particulate material, is conveniently a material such as dicalcium phosphate or lactose.

Incorporation of lower levels of diamorphine for example between 10 and 30% by weight, may necessitate inclusion of low levels of a release modifying component, for example 5 to 15% by weight polyethylene glycol 6000, to achieve a satisfactory in vitro release rate. At higher drug loadings, for example 40 to 75% by weight it is envisaged that only incorporation of very small amounts of polyethylene glycol, for example 0.001 to 1% by weight would be required to modify the in-vitro release rate.

The present invention also provides a process for the manufacture of a sustained release pharmaceutical preparation which, in one aspect, comprises
mechanically working in a high-shear mixer, a mixture of diamorphine or pharmaceutically active salt thereof in particulate form and a particulate, hydrophobic, fusible carrier or diluent having a melting point from 35 to 150°C e.g. to 65°C and optionally a release modifying component comprising a water soluble, fusible material or a particulate, soluble or insoluble organic or inorganic material, at a speed and energy input which allows the carrier or diluent to melt or soften whereby it forms a matrix of hydrophobic, fusible carrier or diluent, containing diamorphine or salt thereof dispersed therein e.g. in the form of multiparticulates or granules or agglomerates.

A preferred process for the manufacture of sustained release multiparticulates or granules or controlled release seeds or particles containing diamorphine or a salt thereof in accordance with the invention comprises
(a) mechanically working in a high-shear mixer, a mixture of diamorphine or salt thereof in particulate form and a particulate, hydrophobic fusible carrier or diluent having a melting point from 35 to 150°C and optionally a release modifying component comprising a water soluble fusible material, or a particulate soluble or insoluble organic or inorganic material at a speed and energy input which allows the carrier or diluent to melt or soften, whereby it forms agglomerates;
(b) breaking down agglomerates from (a) or product from step (c) to give controlled release seeds or particles; and optionally
(c) continuing mechanically working optionally with a further addition of low percentage of the carrier or diluent; and
(d) optionally repeating step (b) and possibly (c) one or more e.g. up to five times.

The agglomerates from step (a) may be large, irregularly shaped agglomerates.

The product from step (c) may be obtained as large irregularly shaped agglomerates, or beads or spherical or spheroidal pellets. As indicated in (d) the product from step (c) may be returned and treated according to step (b) one or more times.

This process is capable of giving a high yield (over 80%) of multiparticulates in a desired size range, with a desired in vitro release rate, and uniformity of release rate.

Resulting multiparticulates granules or controlled release seeds or particles may be sieved or milled to eliminate any oversized or undersized material then formed into the desired dosage units by for example, encapsulation into hard gelatin capsules containing the required dose of the active substance.

The multiparticulates, or the granules, agglomerates or controlled release seeds or particles obtained from the respective steps of the process can, if desired, be compressed, as mentioned above to form controlled release tablets.

Preferably diamorphine or salt thereof is used in an amount which results in multiparticulates containing between 10% and 75%, especially between about 45% and about 75% w/w active ingredient for a high dose product and 10 and 45% for a low dose product.

Usually dosage units containing 10 mg to 1000 mg of diamorphine or salt thereof e.g. 20 mg; 500 mg; 40 mg and 200 mg.

In the preferred method of the invention preferably all the drug is added in step (a) together with a major portion of the hydrophobic, fusible release control material used. Preferably the amount of fusible, release control material added in step (a) is between 25% and 75% w/w e.g between 25% and 45 % w/w of the total amount of ingredients added in the entire manufacturing operation, more preferably between 30% and 60% e.g. between 30% and 40%.

Preferably all the fusible release control material is added at stage (a).

Preferably stage (a) of the process may be carried out in conventional high-shear mixers with a stainless steel interior, e.g. a Collette Vactron 75 or equivalent mixer. The mixture is processed until a bed temperature above 40°C is achieved and the resulting mixture acquires a cohesive granular texture, with particle sizes ranging from about 1-3mm to fine powder in the case of non-aggregated original material. Such material, in the case of the embodiments described below, has the appearance of agglomerates which upon cooling below 40°C have structural integrity and resistance to crushing between the fingers. As this stage the agglomerates are of an irregular size, shape and appearance.

The agglomerates are preferably allowed to cool. The temperature to which it cools is not critical and a temperature in the range room temperature to 45°C e.g. 20°C to 30°C may be conveniently used.

The agglomerates are broken down by any suitable means, which will comminute oversized agglomerates and produce a mixture of powder and small particles preferably with a diameter under 2mm. It is currently preferred to carry out the classification using a Jackson Crockatt granulator using a suitable sized mesh, or a Comil with an appropriate sized screen. We have found that if too small a mesh size is used in the aforementioned apparatus the agglomerates melting under the action of the beater or impeller will clog the mesh and prevent further throughput of mixture, thus reducing yield. A mesh size of 12 or greater or a 094G Comil screen have been found adequate.

In a preferred method the classified material is returned to the high shear mixer and processing continued. It is believed that this leads to cementation of the finer particles into multiparticulates of uniform size range or into large, irregularly shaped agglomerates, which agglomerates may be milled e.g. in a Comil or Jackson-Crocket.

In this process of the invention the temperature of the mixing bowl throughout the mechanical working is chosen so as to avoid excessive adhesion of the material to the walls of the bowl. We have generally found that the temperature should be neither too high nor too low with respect to the melting temperature of the fusible material and it can be readily optimised to avoid the problems mentioned above. The same applied to the process of mechanically working a mixture of drug and particulate hydrophobic fusible carrier in a high speed mixture first mentioned above. For example in the processes described below in the Examples a bowl temperature of approximately 60°C has been found to be satisfactory and avoid adhesion to the bowl.

In order to ensure uniform energy input into the ingredients in the high shear mixer it is preferred to supply at least part of the energy by means of microwave energy.

Energy may also be delivered through other means such as by a heating jacket or via the mixer impeller and chopper blades.

After the multiparticulates, granules or controlled release seeds or particles have been formed they may be sieved, to remove any over or undersized material, and allowed to cool before or after sieving.

The resulting multiparticulates, granules or controlled release seeds or particles may be used to prepare dosage units such as tablet or capsules in manners known per se. To produce tablets in accordance with the invention, multiparticulates granules or controlled release seeds or particles produced as described above may be mixed or blended with the desired excipient(s), if any, using conventional procedures e.g. using a Y-Cone or bin-blender and the resulting mixture compressed according to conventional tabletting procedure using a suitably sized tabletting tooling. Tablets can be produced using conventional tabletting machines, and in the embodiments described below were produced on a standard single punch F3 Manesty machine or Kilian RLE15 rotary tablet machine.

Generally speaking we find that tablets formed by compression according to standard methods give very low in vitro release rates of the active ingredient. We have found that the in vitro release profile can be adjusted in a number of ways. For instance a higher loading of the drug will be associated with increased release rates; the use of larger proportions of a water soluble fusible material in the particulates or surface active agent in the tabletting formulation will also be associated with a higher release rate of the active ingredient. Thus by controlling the relative amount of these ingredients it is possible to adjust the release profile of the active ingredient.

The present invention also comprehends a method of treating a patient suffering from pain by administering to the patient a preparation containing an effective amount of diamorphine or a pharmaceutically acceptable salt in accordance with the invention.

The present invention further comprehends a method of treating a person addicted to diamorphine (heroin), by maintenance therapy, which comprises administering to such a person a preparation containing an effective amount of diamorphine or pharmaceutically acceptable salt thereof in controlled release form according to the present invention.

The administration of the two aforesaid methods of treatment comprises administration by a physician or staff, and self administration.

Such administration will preferably be once, or twice daily.

### Example 1

0.35kg particulate diamorphine hydrochloride and the same weight of particulate hydrogenated vegetable oil (LUBRITAB) were placed in the bowl of a Collette Gral 10 or equivalent mixer, preheated to 60°C. Mixing was carried out at the following speeds for the Collette Gral 10 - mixer 350 rpm; chopper 1500 rpm, until the contents of the bowl were slightly agglomerated. The agglomerates were then allowed to cool to approximately 40°C, and were milled in a Comil to obtain controlled release seeds. The seeds were then placed in the mixer bowl and processing carried out until multiparticulates of a desired size were obtained. The contents of the bowl were then discharged and sieved to collect the 0.5 - 2.0mm sieve fraction.

### Example 2

The procedure of Example 1 was repeated but the collected sieve fraction was blended in a conventional blender with 0.006kg talc for 5 minutes; 0.004kg magnesium stearate is then added and the blending continued for 3 minutes. The blend was then discharged and compressed using a 4mm x 8mm capsule shaped tooling on a F3 tablet machine. The resulting tablet had a hardness of 1.7kp, a thickness of 2.8 - 3.0mm and a friability of < 1.0% w/w and the following constitution.

| **CONSTITUENT** | **MG/TABLET** | **% W/W** |
|---|---|---|
| Diamorphine Hydrochloride | 40.0 | 47.6 |
| Hydrogenated Vegetable Oil | 40.0 | 47.6 |
| Talc | 2.40 | 2.86 |
| Magnesium Stearate | 1.6 | 1.91 |
| TOTAL | 84 | |

### Reference Example 1

The dissolution rates of the resulting multiparticulates and tablets, measured respectively by the Ph. Eur. Basket or Paddle method at 100rpm in either phosphate or acetate buffer, were as follows:

| **TIME (HRS)** | **% DIAMORPHINE HCL RELEASED** | | |
|---|---|---|---|
| | **Multiparticulates Basket/Phosphate Buffer** | **Tablets Paddle/Phosphate Buffer** | **Tablets Paddle/Acetate Buffer** |
| 1 | 30 | - | 24 |
| 2 | 44 | 35 | 35 |
| 3 | 54 | 41 | 43 |
| 4 | 62 | 47 | 49 |
| 6 | 70 | 57 | 59 |
| 8 | 78 | 64 | 67 |
| 12 | 87 | 75 | 78 |
| 16 | 92 | 84 | 86 |

### Reference Example 2

The diamorphine hydrochloride content and the content of related substances was evaluated during storage at 30°C over three months, the storage being in a polypropylene container with a polyethylene lid, with the following result:

| **PRODUCT** | **INITIAL ANALYSIS (mg)** | **3 MONTHS STORAGE(mg)** | **6 MONTHS STORAGE (mg)** |
|---|---|---|---|
| **Diamorphine HCL** | **38.7** | **38.9** | **40.1** |
| **6-0-acetylmorphine HCL** | **0.5** | **0.5** | **0.5** |
| **Morphine HCL** | **0.0** | **0.0** | **0.0** |

The dissolution rate of the tablets was measured by the Ph. Eur. Paddle method at 100rpm in pH 4.5 acetate buffer directly after preparation and after three months storage at 30°C, with the following result:

| **TIME (HRS)** | **% DIAMORPHINE HCl RELEASED** | | |
|---|---|---|---|
| | Initial Analysis | 3 months | 6 months |
| 1 | 24 | 22 | 22 |
| 2 | 36 | 32 | 32 |
| 3 | 44 | 41 | 39 |
| 4 | 50 | 47 | 45 |
| 5 | 55 | 52 | 51 |
| 6 | 59 | 57 | 55 |
| 7 | 64 | 61 | 59 |
| 8 | 68 | 65 | 63 |
| 9 | 70 | 68 | 66 |
| 10 | 73 | 71 | 69 |
| 11 | 76 | 74 | 71 |
| 12 | 79 | 77 | 74 |
| 13 | 81 | 80 | 76 |
| 14 | 83 | 81 | 78 |
| 15 | 86 | 84 | 80 |
| 16 | 87 | 86 | 82 |
| 17 | 89 | 87 | 84 |
| 18 | 91 | 90 | 86 |
| 19 | NR | 91 | 87 |
| 20 | NR | 92 | 89 |

### Comparative Example 1

| **TABLET FORMULATION** | **MG/TABLE T** |
|---|---|
| Diamorphine hydrochloride | 10.0 |
| Lactose anhydrous | 90.0 |
| Hydroxyethylcellulose - (Natrosol 250HX) | 10.0 |
| Purified Water | q.s. |
| Certostearyl alcohol (Dehydagwax) | 35.0 |
| Talc | 3.0 |
| Magnesium Stearate | 2.0 |
| **TOTAL** | 150.0 |

The diamorphine hydrochloride, lactose and hydroxyethyl cellulose were blended in a Collette Gral high speed mixer or equivalent. Water was added and the powder blend granulated by operating the mixer.

The resulting granulate was partially dried in fluid bed drier with an inlet air temperature of 60°C. The partially dried granulate was passed through a 12 mesh screen, then completely dried and passed through a 16 mesh screen.

The granules, whilst still warm were blended with molten cetostearyl alcohol at 65°C using a mixer.

The resulting granules were cooled and passed through a 16 mesh screen, blended with the appropriate amounts of talc and magnesium stearate using a suitable blender and compressed into tablets on a suitable tableting machine using 7.14mm diameter deep concave tooling.

The dissolution rate of the resulting tablets, measured by USP paddle at 150rpm in distilled water was:

| **TIME (HRS)** | **% DIAMORPHINE RELEASED** |
|---|---|
| 1 | 54 |
| 2 | 71 |
| 3 | 83 |
| 4 | 89 |
| 5 | 95 |
| 6 | 99 |
| 7 | 100 |

The diamorphine hydrochloride content of the tablets was evaluated during storage at room temperature, 30°C and 30°C/80% relative humidity with the following results;

| **Storage Time (Months)** | **Storage Temperature** | | |
|---|---|---|---|
| | **Diamorphine HCI content (mg/tablet)** | | |
| | **Room Temperature** | **30°C** | **30°C/80% RH** |
| 0 | 10.2 | - | - |
| 1 | 10.1 | 9.5 | 9.4 |
| 3 | 10.6 | 9.8 | 9.8 |
| 6 | 9.5 | 7.0 | 6.3 |

### Comparative Example 2

| **Tablet formulation** | **Mg/tablet** |
|---|---|
| Diamorphine hydrochloride | 30.0 |
| Lactose anhydrous | 70.0 |
| Hydroxyethylcellulose - (Natroxol 250HX) | 10.0 |
| Purified Water | q.s. |
| Cetostearyl alcohol (Dehydagwax) | 35.0 |
| Talc | 3.0 |
| Magnesium Stearate | 2.0 |
| **TOTAL** | 150.0 |

The tablets were prepared from the above constituents and tested using the procedures described in Comparative Example 1.

The dissolution rates observed were as follows:

| **TIME (HRS)** | **% DIAMORPHINE RELEASED** |
|---|---|
| 1 | 52 |
| 2 | 68 |
| 3 | 81 |
| 4 | 91 |
| 5 | 96 |
| 6 | 99 |
| 7 | 100 |

The diamorphine hydrochloride content of the tablets under the various storage conditions was found to be as follows:

| **Storage Time (Months)** | **Storage Temperature** | | |
|---|---|---|---|
| | **Diamorphine HCl content (mg/tablet)** | | |
| | **Room temperature** | **30°C** | **30°C/80% RH** |
| 0 | 33.2 | - | - |
| 1 | 30.7 | 31.4 | 29.6 |
| 3 | 30.3 | 30.0 | 30.1 |
| 6 | 29.4 | 28.2 | 28.3 |

It can be seen from the formulations of diamorphine in accordance with the invention provide for a controlled release for twice or once-a-day dosing - which is not possible with formulation of diamorphine in a conventional controlled release matrix.

The foregoing examples also show that formulations of the invention have unexpectedly superior storage stability compared with a formulation using the conventional controlled release matrix both in terms of stability of the absolute quantity of active ingredient and degradation products, and the in vitro release rates of diamorphine or salt.

## Claims

1. A controlled release, pharmaceutical preparation preferably for oral administration which comprises diamorphine or a pharmaceutically acceptable salt thereof as an active ingredient.

2. A preparation according to Claim 1 which comprises of a matrix comprising a hydrophobic, fusible carrier or diluent in which diamorphine or a salt thereof is incorporated.

3. A preparation according to Claim 2, in the form of multiparticulates, granules or controlled release seeds comprising diamorphine or a pharmaceutically acceptable salt thereof incorporated in a controlled release matrix.

4. A unit, oral dosage form which comprises a capsule containing multiparticulates or granules as set forth in Claim 2 or Claim 3 and optionally conventional capsuling excipients.

5. A tablet comprising compressed multiparticulates, granules or controlled release seeds according to Claim 2 or Claim 3, and optionally conventional tabletting excipients.

6. A controlled release, pharmaceutical preparation according to any one of the preceding claims which has a dissolution rate obtained by a method as herein before defined, as follows:
| **TIME (HR)** | **% DIAMORPHINE RELEASED** |
|---|---|
| 1 | 0-70 |
| 2 | <80 |
| 4 | 10-100 |
| 12 | 40-100 |
| 16 | 50-100 |

7. A preparation according to claim 6, wherein the release rate is as follows:
| **TIME (HR)** | **% DIAMORPHINE RELEASED** |
|---|---|
| 1 | 10 - 70 |
| 2 | 15 - 80 |
| 4 | 30 - 100 |
| 6 | 40 - 100 |
| 12 | 60 - 100 |
| 16 | 70 - 100 |

8. A preparation according to claim 6, wherein the release rate is as follows:
| **TIME (HR)** | **% DIAMORPHINE RELEASED** |
|---|---|
| 1 | 0-50 |
| 4 | 10-80 |
| 8 | 30-100 |
| 12 | 40-100 |
| 16 | 50-100 |

9. A preparation according to any one of the preceding claims, which contains an amount of diamorphine or salt thereof sufficient for up to 24 hours dosing, and which provides for controlled release of the diamorphine or salt such that it is suitable for 12 hourly, preferably 24 hourly administration.

10. A process for the manufacture of a sustained release pharmaceutical preparation which comprises mechanically working in a high shear mixer, a mixture of diamorphine or pharmaceutically active salt thereof in particulate form and a particulate, hydrophobic, fusible carrier or diluent having a melting point from 35 to 150°C e.g. to 65°C and optionally a release modifying component comprising a water soluble, fusible material or a particulate, soluble or insoluble organic or inorganic material, at a speed and energy input which allows the carrier or diluent to melt or soften whereby it forms a matrix of hydrophobic fusible carrier or diluent containing diamorphine or salt thereof dispersed therein.

11. A process for the manufacture of sustained release multiparticulates, granules or controlled release seeds containing diamorphine or a salt thereof which comprises.
(a) mechanically working in a high-shear mixer, a mixture of diamorphine or salt thereof in particulate form and a particulate, hydrophobic, fusible carrier or diluent having a melting point from 35 to 150°C e.g. to 100°C and optionally a release modifying component comprising a water soluble fusible material, or a particulate soluble or insoluble organic or inorganic material, at a speed and energy input which allows the carrier or diluent to melt or soften, whereby it forms agglomerates;
(b) breaking down from step (a) or products from step (c) agglomerates to give controlled release seeds and particles;
and optionally
(c) continuing mechanically working optionally with a further addition of low percentage of the carrier or diluent; and
(d) optionally repeating step (b) and possibly (c) one or more e.g. up to five times.

12. A process according to Claims 10 or 11 wherein the product is sieved or milled to obtain multiparticulates, granules or controlled release seeds or particles of a desired size range.

13. A process according to Claim 12, wherein the multiparticulates or granules are further processed by filling into capsules or the multiparticulates, granules or controlled release seeds or particles are compressed to form tablets, optionally after mixing respectively with conventional capsulling or tabletting excipients.

14. A method of treating a patient suffering from pain which comprises administering to he patient a preparation according to any one of claims 1 to 9 containing an effective amount of diamorphine or a pharmaceutically acceptable salt thereof.

15. A method of treating a heroin addict by maintenance therapy, which comprises administering to the addict a preparation according to any one of claims 1 to 9, containing an effective amount of diamorphine or a pharmaceutically acceptable salt thereeof.
